# EUROPEAN PATENT APPLICATION

(11) **EP 3 305 281 A1**
(43) Date of publication of application: **11.04.2018**
(21) Application number: 17187813.5
(22) Date of filing: 17.05.2010
(51) Int. Cl.: A61K 9/08, A61K 47/32, A61K 31/4174, A61P 29/00

(54) **SUBLINGUAL DEXMEDETOMIDINE COMPOSITIONS AND METHODS OF USE THEREOF**

(30) Priority: 15.05.2009 US 178730 P
(62) Divisional of application: 10775671.0
(71) Applicant: Recro Pharma, Inc., Malvern PA 19355 (US)
(72) Inventor: HENWOOD, Geraldine Anne, Malvern, PA 19355 (US); MACK, Randall Jerome, Paoli, PA 19301 (US); SHARR, Christopher Thomas, Malvern, PA 19355 (US); KOLENG, Jr., John Joseph, Austin, TX 78750 (US)
(74) Representative: Bassil, Nicholas Charles

(57) **Abstract**

Analgesic sublingual formulations of dexmedetomidine and methods of use thereof are provided for use in the prevention, treatment and management of pain and other conditions.

## Description

### FIELD

This application describes analgesic, sublingual-spray formulations of dexmedetomidine, pharmaceutically acceptable salts thereof, and derivatives thereof, as well as methods of use thereof.

### BACKGROUND

Dexmedetomidine, 5-[(1*S*)-1-(2,3-dimethylphenyl)ethyl]-1*H*-imidazole, is a non-narcotic α₂-adrenoceptor agonist with sedative and analgesic properties.

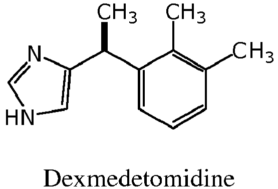

Currently, dexmedetomidine is only commercially available as an injectable formulation indicated for sedation, and it must be administered intravenously by a heath care professional. Although dexmedetomidine has analgesic properties, a formulation useful as an analgesic, however, is not commercially available. Moreover, for a variety of reasons the commercially available injectable formulation is not suitable for use as an analgesic that can be self-administered. A continuing and unmet need exists for a dexmedetomidine-based analgesic medicines that, for example, may be self-administered to produce analgesia (or otherwise treat or prevent pain) without significant sedation.

### BRIEF SUMMARY

Provided herein are new analgesic, sublingual-spray formulations of dexmedetomidine and/or a pharmaceutically acceptable salt thereof, and/or a derivative thereof, and methods of use thereof in the treatment or prevention of pain. Such pharmaceutical compositions include dexmedetomidine or a pharmaceutically acceptable salt or derivative (e.g., pro-drug) thereof, such as in an amount sufficient to produce analgesia (e.g., treat or prevent pain) and a pharmaceutically acceptable liquid vehicle, as well as optional flavoring agents, pressurized propellants, preservatives, excipients, emulsifiers, buffers, colorants, and the like.

In an example, a method is provided for treating or preventing pain comprising the steps of applying to the oral mucosa of a human a pharmaceutical composition comprising a dosage of dexmedetomidine or a pharmaceutically acceptable salt thereof in a pharmaceutically acceptable liquid vehicle, wherein the dexmedetomidine or pharmaceutically acceptable salt thereof is absorbed through said oral mucosa and produces analgesia without sedation. In another example, the dosage of dexmedetomidine or pharmaceutically acceptable salt thereof is between about 0.05 µg/kg and about 1.50 µg/kg. By further example, the method is utilized wherein the human is an adult, and the dosage of dexmedetomidine or pharmaceutically acceptable salt thereof is between about 5 µg and about 50 µg. Still further, the method may provide that the plasma Cₘₐₓ of dexmedetomidine upon transmucosal absorption into the systemic circulatory system of said human is less than about 0.30 ng/mL.

In another example, a method is provided for treating or preventing pain. In this example, the steps include administering to the oral mucosa of a mammal a systemically absorbed pharmaceutical composition comprising dexmedetomidine, or a pharmaceutically acceptable salt or pro-drug thereof, in an amount effective to treat or to prevent pain in said mammal upon administration, wherein the pharmaceutical composition provides a physiologically active amount of dexmedetomidine into the systemic circulatory system of said mammal at a rate that produces an analgesic effect without sedation within one hour of administration.

In yet another example, an analgesic pharmaceutical composition is provided comprising dexmedetomidine, or a pharmaceutically acceptable salt or pro-drug thereof, in and a pharmaceutically acceptable liquid vehicle, said pharmaceutical composition being configured and adapted for transmucosal administration to a mammal by applying said analgesic pharmaceutical composition to a mucous membrane of said mammal. Still further, the analgesic pharmaceutical composition may be configured and adapted for sublingual administration by applying said composition to a mucous membrane under the tongue of said mammal.

In yet another an exemplary embodiment, a method for administering dexmedetomidine or a pharmaceutically acceptable salt or derivative thereof to a mammal includes spraying the oral mucosa of the mammal with a metered dosage of a spray composition comprising dexmedetomidine or a pharmaceutically acceptable salt or derivative thereof in a pharmaceutically acceptable liquid vehicle in an effective amount to provide transmucosal absorption of a pharmaceutically effective amount of the dexmedetomidine through the oral mucosa of the mammal into the systemic circulatory system of the mammal. Upon transmucosal absorption, the dexmedetomidine produces analgesia in the mammal.

Additional features may be understood by reference to the following detailed description and examples.

### DETAILED DESCRIPTION

Provided herein are new analgesic, sublingual-spray formulations of dexmedetomidine or pharmaceutically acceptable salts or derivatives thereof and methods of use thereof in the prevention, treatment, and management of pain.

Dexmedetomidine is a specific α2-adrenergic receptor agonist that causes sedation, anesthesia, and analgesia in mammals. In humans, dexmedetomidine is commercially available for sedation of initially intubated and mechanically ventilated patients during treatment in an intensive care setting, as well as sedation of non-intubated patients prior to or during surgical and other procedures. See, e.g., U.S. Pat. Nos. 6,716,867 and 6,313,311.

The pharmacokinetics of dexmedetomidine in humans have been studied after intravenous (i.v.), intramuscular (i.m.), and transdermal administration. The mean elimination half-life is 1.5 to 3 h after i.v. and i.m. dosing, respectively, and 5.6 h after transdermal administration. After i.m. and transdermal administration, the time to maximum concentration in blood is 1.6 - 1.7 h and 6 h, respectively, and the absolute bioavailability has been estimated to be 73% and 88%, respectively. See, e.g., "Pharmacodynamics and pharmacokinetics of intramuscular dexmedetomidine," Scheinin et al., Clin. Pharmacol. Ther. 52, 53-46 (1992); "The pharmacokinetics and hemodynamic effects of intravenous and intramuscular dexmedetomidine hydrochloride in adult human volunteers," Dyck et al., Anesthesiology 78, 813-20 (1993); and "Pharmacokinetics and pharmacodynamics of transdermal dexmedetomidine," Kivistö et al., Eur. J. Clin. Pharmacol. 46, 345-49 (1994).

Dexmedetomidine is also absorbed from the oral cavity. After buccal administration in which human subjects held a solution of dexmedetomidine in the mouth without swallowing, the mean buccal bioavailability has been measured at 81.8%, with a maximum concentration at approximately 1.5 h and an apparent elimination half-life of 1.9 h. See, e.g., "Bioavailability of dexmedetomidine after extravascular doses in healthy subjects," Anttila et al., Br. J. Clin. Pharmacol. 56, 691-93 (2003).

According to the present invention, dexmedetomidine may be administered to an animal or human subject for the purpose of ameliorating, managing, curing, preventing, or otherwise treating pain. In an exemplary embodiment, a method for administering dexmedetomidine or a pharmaceutically acceptable salt or derivative thereof to a mammal includes spraying the oral mucosa of the mammal with a metered dosage of a spray composition comprising dexmedetomidine or a pharmaceutically acceptable salt or derivative thereof in a pharmaceutically acceptable liquid vehicle in an effective amount to provide transmucosal absorption of a pharmaceutically effective amount of the dexmedetomidine through the oral mucosa of the mammal into the systemic circulatory system of the mammal. Upon transmucosal absorption, the dexmedetomidine produces analgesia in the mammal.

For example, many patients with cancer and other ailments continue to experience moderate to severe pain despite chronic analgesic therapy, and this can occur as intermittent breakthrough pain often due to increases in a patient's activity level. Attempts to counteract this type of pain by increasing the dose of long-acting formulations of analgesics often produce slow onset of analgesia and unwanted side-effects of sedation, constipation or nausea and vomiting, especially with opioid analgesics. However, the analgesic, sublingual-spray formulations of dexmedetomidine described herein selectively provide moderate to rapidly acting, potent non-narcotic analgesics that ameliorates, manages, cures, prevents, or otherwise treats such pain.

The dexmedetomidine products described herein are pharmaceutical formulations for the treatment of pain. As used herein, the term "pharmaceutically acceptable" includes those compounds, materials, compositions, dosage forms, and methods of use thereof that are within the scope of sound medical judgment and suitable for use in contact with the tissues of human beings and animals without excessive toxicity, irritation, allergic response, or other problem or complication, while being commensurate with a reasonable benefit/risk ratio and eliciting a desired pharmacological response.

Dexmedetomidine contains a basic nitrogen atom capable of forming pharmaceutically acceptable salts with pharmaceutically acceptable acids. The term "pharmaceutically acceptable salts" in this respect refers to the relatively non-toxic, inorganic, and organic acid addition salts of dexmedetomidine. These salts may be prepared in situ during final isolation and purification of dexmedetomidine or by separately reacting purified dexmedetomidine in its free base form with a suitable organic or inorganic acid, and thereafter isolating the salt thus formed. Furthermore, the salt may be formed during a manufacturing process to produce the spray formulation. Representative pharmaceutically acceptable salts include the hydrohalide (including hydrobromide and hydrochloride), sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, 2-hydroxyethylsulfonate, and laurylsulphonate salts, and the like. See, e.g., "Pharmaceutical Salts," Berge et al., J. Pharm. Sci. 66, 1-19 (1977). Dexmedetomidine hydrochloride is an example of a pharmaceutically acceptable salt. Use of dexmedetomidine hydrochloride may be preferable to the use of dexmedetomidine per se in the spray formulations described herein because, in some cases, the hydrochloride salt has greater water solubility and stability against oxidation by ambient oxygen.

Dexmedetomidine derivatives may include covalent modifications that create a pro-drug. Upon administration, the pro-drug derivative undergoes chemical modification by the mammal that yields dexmedetomidine. Pro-drugs may be used to favorably alter the biodistribution or the pharmacokinetics of dexmedetomidine or to produce other desirable characteristics. For example, a reactive nitrogen of dexmedetomidine may be derivatized with a functional group that is cleaved, enzymatically or non-enzymatically, reductively, oxidatively, or hydrolytically, to reveal the active pharmaceutical ingredient. Uses of certain types of pro-drugs are known (see, e.g., R.B. Silverman, 1992, "The Organic Chemistry of Drug Design and Drug Action," Academic Press, Chp. 8). For example, pro-drugs may be prepared in situ during the final isolation and purification of the compounds, or by separately reacting the purified compound in its free base form with a suitable derivatizing agent.

The dexmedetomidine spray compositions include one or more pharmaceutically acceptable liquids (from about 30% to about 99.995% by weight). These liquids may be solvents, cosolvents, or non-solvents for dexmedetomidine or its pharmaceutically acceptable salts or derivatives thereof. Suitable materials are liquids at room temperature and remain in the liquid state at room temperature, preferably at both ambient pressure as well as under elevated pressure. Useful liquids are not particularly restricted, provided they do not interfere with the desirable medical use of the spray compositions, and they carry a therapeutically useful amount of dexmedetomidine or a pharmaceutically acceptable salt or derivative thereof (e.g., dexmedetomidine hydrochloride). Examples of pharmaceutically acceptable liquids include water, ethanol, dimethyl sulfoxide, propylene glycol, polyethylene glycol, propylene carbonate, pharmaceutically acceptable oils (e.g., soybean, sunflower, peanut, etc.) and the like. The pharmaceutically acceptable liquid is selected either to dissolves the active pharmaceutical ingredient, to produce a stable, homogenous suspension of it, or to form any combination of a suspension or solution.

In addition to the foregoing constituent ingredients, sublingual-spray formulations of dexmedetomidine may include one or more excipients other than the pharmacologically active drug, which are included in the manufacturing process or are contained in a finished pharmaceutical product dosage form. Examples of excipients include viscosity modulating materials (e.g. polymers, sugars, sugar alcohols, gums, clays, silicas, and the like (e.g., polyvinylpyrrolidone (PVP)) (from about 0.01% to about 65% by weight). Other examples of excipients include preservatives (e.g., ethanol, benzyl alcohol, propylparaben and methylparaben) (from about 0.001% to about 10% by weight). Excipients may also be flavoring agents, sweeteners (e.g., sugars (sucrose, glucose, dextrose, maltose, fructose, etc.), artificial sweeteners (saccharin, aspartame, acesulfame, sucralose), or sugar alcohols (mannitol, xylitol, lactitol, maltitol syrup)) (from about 0.001% to about 65% by weight). Still other examples of excipients include buffers and pH-adjusting agent (e.g., sodium hydroxide, citrate, and citric acid) (from about 0.01% to about 5% by weight). Coloring agents (from about 0.001% to about 5% by weight), fragrances (from about 0.001% to about 1% by weight), chelating agents (e.g., EDTA) (from about 0.001% to about 1% by weight), UV absorbers (from about 0.001% to about 10% by weight), and antifoam agents (e.g., low molecular weight alcohols, dimethicone, simethicone) (from about 0.001% to about 5% by weight), among others, are additional examples of suitable excipients.

Sublingual dexmedetomidine formulations (such as sprays, drops, and the like) may be made by mixing appropriate quantities of the foregoing ingredients in accordance with standard good manufacturing practices. Such excipients may be included in the formulation to improve patient or subject acceptance or taste, to improve bioavailability, to increase shelf-life, to reduce manufacturing and packaging costs, to comply with requirements of governmental regulatory agencies, and for other purposes. The relative amounts of each ingredient should not interfere with the desirable pharmacological and pharmacokinetic properties of the resulting formulation.

The analgesic, sublingual- formulations of dexmedetomidine described herein are intended for administration directly to the mucosa (e.g., the oral mucosa in a mammal). Drug delivery occurs substantially via the oral transmucosal route and not via swallowing followed by gastrointestinal absorption. The term "transmucosal" refers to delivery across or through a mucosal membrane. In particular, "oral transmucosal" delivery of a drug includes delivery across any tissue of the mouth, pharynx, larynx, trachea, or upper gastrointestinal tract, particularly the sublingual, buccal, gingival and palatal mucosal tissues.

The term "sublingual" literally means "under the tongue" and refers to a method of administering substances via the mouth in such a way that the substances are rapidly absorbed via the blood vessels under the tongue rather than via the digestive tract. Sublingual absorption occurs through the highly vascularized sublingual mucosa, which allows a substance direct access to the blood circulation, thereby providing for direct systemic administration independent of gastrointestinal influences and avoiding undesirable first-pass hepatic metabolism. As compared to other routes of administration, transmucosal absorption of dexmedetomidine in the present formulations may have a significantly faster onset with greater bioavailability. Accordingly, the total amount of active pharmaceutical ingredient in the formulation may be reduced, thereby reducing the likelihood of deleterious side effects and providing a cost benefit to the manufacturer.

The formulation may be administered to mammals, including humans, as well as human companion animals (e.g., cats, dogs), agricultural livestock, and other animals in need thereof. One will appreciate that administering conventional dosage forms such as tablets, capsules, syrups, etc. or injectable analgesic formulations to non-human animals is often problematic, and the sublingual spray formulations described herein are especially useful in the treatment of such animals,

"Analgesia" is the alleviation or elimination of the sensation of pain. As used herein, "pain" encompasses a wide range of clinical manifestations, and it has a broad meaning. Pain perception is highly subjective, and different people experience pain in different ways and with greatly different intensities. The International Association for the Study of Pain defines pain as "an unpleasant sensory and emotional experience associated with actual or potential tissue damage, or described in terms of such damage." More simply stated, pain includes any sensory experience that causes suffering and is associated with an unpleasant awareness of one's own body. Non-limiting types and causes of pain include neuralgia, myalgia, hyperalgesia, hyperpathia, neuritis, and neuropathy. Pain is often a symptom of an underlying physiological abnormality, such as cancer or arthritis. Some types of pain have no clearly identified causes, such as migraine headache pain. Pain may also be caused by physical trauma, such as burns or surgery. Viral infections, such as Herpes zoster (chicken pox and shingles), can also cause pain. Withdrawal from chemical dependence on alcohol or drugs of abuse is also often associated with pain symptoms. Accordingly, "pain" is understood herein to have a very broad meaning and it's claimed uses should not be construed as being limited to any particular malady or condition.

"Sedation" as used herein means depressed consciousness in which a patient or subject retains the ability to independently and continuously maintain an open airway and a regular breathing pattern, and to respond appropriately and rationally to physical stimulation and verbal commands. As used herein "without significant sedation" means that the Patient experiences a level of sedation not greater than Level 3 on the Ramsay Sedation Scale, in other words, the Patient is either at Level Level 1 = anxious, agitated, or restless; Level 2 = cooperative, oriented, and tranquil; or Level 3 = sedated but responds to commands. "Significant sedation" as used herein means that the patient or subject experiences sedation of Level 4 or greater on the Ramsay Sedation Scale, wherein Level 4 = asleep; brisk response to light glabellar tap or loud auditory stimulus; Level 5 = asleep; sluggish response to light glabellar tap or loud auditory stimulus; Level 6 = asleep; no response to painful stimulus. "Significant sedation" as used herein is also consistent with a patient's self-evaluation on the Stanford Sleepiness Scale, with Subject patients rating their degree of sedation as greater than or equal to Level 3, wherein: Level 1 = Feeling active, vital, alert, or wide awake; Level 2 = Functioning at high levels, but not at peak; able to concentrate; Level 3 = Awake, but relaxed; responsive but not fully alert; Level 4 = Somewhat foggy, let down; Level 5 = Foggy; losing interest in remaining awake; slowed down; Level 6 = Sleepy, woozy, fighting sleep; prefer to lie down; or Level 7 No longer fighting sleep, sleep onset soon; having dream-like thoughts.

The sublingual- formulations of dexmedetomidine described herein may be co-administered with other pain-treatment medicines, including NSAIDS such as aspirin, ibuprofen, naproxen, celecoxib, acetaminophen, and other cyclooxygenase inhibitors; opioids such as codeine, oxycodone, morphine, methadone, and fentanyl; anticonvulsants and antiarrhythmics such as phenytoin and carbamazepine; and antidepressants such as amitriptyline, imipramine, venlafaxine, clonidine and other active α-2 receptor agonist compounds. Such co-administration may be contemporaneous, wherein dexmedetomidine and another pain-treatment medicine are both administered at the same time. Alternatively, because of the selectively moderate to rapid-acting nature of the sublingual compositions described herein, a patient may be administered a longer acting pain medicine on a regular schedule, with sublingual-spray dexmedetomidine being administered as needed throughout the day or from time to time as required. In some cases, the dosage of the longer acting pain-treatment medicine may be reduced because of a beneficial synergistic effect produced by dexmedetomidine, which supplements the primary pharmacological therapy. In particular, dexmedetomidine may significantly potentiate the effectiveness of opioids, permitting a reduction in required opioid dosage while maintaining equivalent therapeutic usefulness.

Analgesic, sublingual-spray formulations (or other sublingual and/or buccal formulations herein) of dexmedetomidine or a pharmaceutically acceptable salt or derivative thereof are preferably provided in metered dosages so that a predetermined amount of the active pharmaceutical ingredient is properly administered to the subject in a pharmaceutically effective amount. For example, the sublingual-spray formulations may be packaged as a bulk liquid containing multiple doses in a pump spray system comprising a sealed container fitted with a metering pump. Typically, a human patient is treated by sublingual self-administration, such as by of one or more actuations from a spray pump. An advantage of sublingual-spray delivery examples herein is the ability to titrate patients by single doses as required through single, discrete actuations. This advantage is typically absent from other forms of drug delivery (e.g., patches, lozenges, tablets, and suppositories) in which a one-size-fits-all dosage is administered in a standard regimen. Additional advantages of sublingual spray formulations include their ease of use, especially when self-administered absent an attending health care professional.

Pump action sprays are characterized in requiring the application of external pressure for actuation, for example, external manual, mechanical or electrically initiated pressure. This is in contrast to pressurized systems, e.g., propellant-driven aerosol or compressed gas sprays, where actuation is typically achieved by controlled release of pressure e.g., by controlled opening of a valve. In certain embodiments, pump sprays are preferred as the use of a pump spray with the formulations herein allows for the administration of droplets or particles having a small mean diameter and a controllable size distribution of droplets. In other embodiments, pressurized systems containing a reservoir of pressurized propellant gas (e.g., carbon dioxide, nitrogen, chlorofluorocarbons, hydrofluoroalkanes, etc.) may produce suitable particles or droplets. Liquid droplets or particles having a diameter that is too small have the potential to enter into the lungs of a human upon administration. In certain preferred embodiments, the droplet size of the delivered formulations further provides for an increase in surface area by being sprayed sublingually as opposed to being placed under the tongue, e.g., with a dropper. The size of the spray particles and shape of the spray pattern also may contribute to whether the active ingredient is absorbed into body systems other than the oral mucosa (e.g., lungs).

The spray pump device may be premetered or, alternatively, the device may be device-metered. Premetered devices preferably contain previously measured doses or a dose fraction in some type of units (e.g., single unit dose amount of solution, single or multiple blisters or other cavities) that may be included in the device during manufacture or by the patient before use. Typical device-metered units have a reservoir containing formulation sufficient for multiple doses that are delivered as metered sprays by the device itself when activated by the patient. The device may be metered both in the amount of drug substance delivered (i.e., the dosage per actuation), as well as the length of time between each dosage. Limiting the time between each dosage can prevent over-use by limiting how often a dosage can be delivered to the patient.

Manufacturing considerations include the reproducibility of the dose, the spray plume, and the particle/droplet size distribution, which can affect the delivery of the dexmedetomidine, pharmaceutically acceptable salt thereof, or derivative thereof, under the tongue. Maintaining the reproducibility of these parameters through the expiration dating period and ensuring the functionality of the device (e.g., spray mechanism, electronic features, sensors, and the like) through its lifetime under patient-use conditions is important as any alteration in these parameters could lead to variability in dosing and absorption, which could lead to potential side effects and decreased therapeutic usefulness.

The administered dose of spray formulation may be dependent on the design, reproducibility, and performance characteristics of the container closure system. A suitable device that provides the desired droplet/particle size distribution is an important factor for the correct performance of the dexmedetomidine product. Actuation parameters (e.g., force, speed, hold and return times) should also be considered with respect to the device. Moreover, the device should be compatible with formulation components. Furthermore, the device should be designed to prevent partial metering, as well as over metering, of the dexmedetomidine formulation, including the dexmedetomidine, pharmaceutically acceptable salt thereof, or derivative thereof, when used according to patient instructions for use.

A typical spray delivery device includes a base unit, a discharge actuator, an orifice for the formulation to be release from the device, and a reservoir. Preferably the reservoir is filled with the drug substance and other excipients (e.g., liquid vehicle, flavors, sweetners, etc. as discussed elsewhere herein) prior to dispensing to the patient, e.g., at the manufacturing site. The reservoir preferably defines a measured amount of dexmedetomidine, pharmaceutically acceptable salt thereof, or derivative thereof to be discharged upon activation. The reservoir body may be any acceptable material, for example, formed simply by a section of a cylindrical hollow of a plastic, steel, such as stainless steel, transparent material, or the like so that its production is very simple. An actuator, which is movable relative to the orifice for activating discharge, may be provided on or with the device. In the course of the actuating movement, the reservoir opens, e.g. by puncturing, to administer a single dosage through an orifice. During a part of the actuating travel following the starting position an elevated pressure is built up. In a subsequent portion of the actuating movement continuing in the same direction, the medium may be relieved of the pressure at one of the sides and communicated to an orifice. In such a manner, the medium is pushed from the reservoir and through the orifice by the action of pressure.

Typically as the liquid formulation leaves the orifice, the liquid droplets follow a trajectory which is influenced by the orifice shape, as well as by pressure asserted. In some embodiments, the droplet size, spray geometry, and the spray pattern are dependent on the design of the pump and/or the properties of the formulation. In certain embodiments, the orientation of the actuator, pump design, and the properties of the formulation will influence the spray symmetry and the shape. The spray pattern may also be optimized to disperse the droplets over a wider pathway thereby increasing the surface area through which the compound can be absorbed and reducing the swallowing reflex. The spray device may further be designed to facilitate ease of patient use and placement of the administered spray to specific regions of the oral mucosa.

The previous preferred spray embodiment is not intended as limiting. In practicing the invention, formulations containing dexmedetomidine may alternatively or additionally be provided as other sublingual and/or buccal compatible dosage forms. For example, sublingual liquid provided as a liquid compatible with administration by a dropper or similar device are contemplated by the inventors. In another example, the sublingual formulation can be packaged in pharmaceutically acceptable unit dose ampules with snap-off tops to permit the opened ampule to be tipped under a patient's tongue to dispense a single dose of the formulation.

### EXAMPLES

The following sublingual formulations of dexmedetomidine were prepared by mixing the components listed in Table 1. The relative amounts of each component are listed by weight.

**Table 1.**

| Ingredient | Formulation Trial No. "29" | Formulation Trial No. "27" | Formulation Trial No. "28" |
|---|---|---|---|
| Dexmedetomidine●HCl | 0.0295% (w/w) | | |
| Povidone | 2.00 | 4.00 | 2.00 |
| Propylparaben | 0.02 | 0.02 | 0.02 |
| Methylparaben | 0.18 | 0.18 | 0.18 |
| Maltitol Syrup | 25.00 | 24.00 | 25.00 |
| Anhydrous Citric Acid | 0.026 | (none) | (none) |
| Sodium Citrate●2H₂O | 0.257 | (none) | (none) |
| Sodium Hydroxide (1 N) | (none) | QS to desired pH** | QS to desired pH** |
| Ethanol (190 proof) | 2.00 | 2.00 | 2.00 |
| Purified Water, USP | 70.49 | QS | QS |

| | | | |
|---|---|---|---|
| ** Desired pH is apprx. 6.5 to 7.0, which is slightly more acidic than the pKa of protonated dexmedetomidine (pKa = 7.1). | | | |

The formulations described in Table 1 were tested in mammals, as described herein below. The physical properties of the three formulations in Table 1 are described in Table 2, also below.

**Table 2.**

| Formulation Trial No. | Appearance | pH | Specific gravity (g/mL) | Viscosity (cP) |
|---|---|---|---|---|
| "27" | Clear, slightly pale yellow liquid | 6.6 | 1.070 | 4.05 |
| "28" * | Clear, colorless liquid | 6.6 | 1.075 | 2.89 |
| "29" ** | Clear, colorless liquid | 6.2 | 1.078 | 2.63 |

The purpose of the study was to assess the pharmacokinetics and absolute bioavailability of dexmedetomidine in a group of five Beagle dogs (males, each aged 5 to 8 months and weighing between 7.5 and 9.2 kg) following sublingual spray administration of the different formulations described in Tables 1 and 2. Ethical treatment of animals was in accordance with the principles outlines in the USDA Animal Welfare Act (9 C.F.R., parts 1, 2 and 3) and the conditions specified in the Guide for the Care and Use of Laboratory Animals (ILAR publication, 1996, National Academy Press), with due regard to the welfare of the subjects balanced against the potential for advancement of animal and human healthcare.

For sublingual administration, a spray application was administered by one depression of a pump device into the oral cavity under the tongue. In order to ensure proper pump priming, two actuations were performed prior to dosing. Following dose administration, serial blood samples were collected form each subject animal by venipuncture of a jugular vein as follows: 0 (predose), 0.083, 0.167, 0.25, 0.33, 0.5, 1, 2, and 6 hours post-sublingual dose. All sublingual dosings showed no adverse events. Following sublingual dose administration, animals appeared normal at all times post-dose. Blood samples were collected in vacutainer tubes containing K₂EDTA as anticoagulant, and derived plasma samples were stored frozen until analyzed.

Data analysis based on subject weight, dosing amount, clinical observations, and blood sampling times revealed that sublingually administered dexmedetomidine has a Cmax = 0.914 ng/mL and Tmax = 20 min

The following sublingual spray formulations of dexmedetomidine were also prepared by mixing the components listed in Table 3 to provide 50 µg dexmedetomidine per 100 µL. The amounts of each component are listed by weight (g), and the resulting formulations were 0.05% (w/w) dexmedetomidine.

**Table 3.**

| Ingredient | Formulation B Trial No. "33" | Formulation A Trial No. "34" |
|---|---|---|
| Dexmedetomidine●HCl | 0.594 | 0.594 |
| Povidone, USP | 40.0 | 20.0 |
| Propylparaben, NF | 0.2 | 0.2 |
| Methylparaben, NF | 1.8 | 1.8 |
| Maltitol Syrup | 240 | 250 |
| Ethanol, USP (190 proof) | 20 | 20.0 |
| Anhydrous Citric Acid, USP | (none) | 0.26 |
| Sodium Citrate Dihydrate, USP | (none) | 2.57 |
| Purified Water, USP (part 1) | 670 | 680.0 |
| Sodium Hydroxide, NF (4% w/w) | QS pH 7 | (none) |
| Purified Water, USP (part 2) | QS to 1000 g | QS to 1000 g |
| Additional Reference (Clinical) | DEX-SL.02 | DEX-SL.01 |

The physical properties of the two formulations in Table 3 are described in Table 4, below.

**Table 4.**

| Formulation | Trial No. | Appearance | pH | Viscosity (cP) |
|---|---|---|---|---|
| B | 33 | Clear, very slightly yellow liquid | 7.2 | 4.34 |
| A | 34 | Clear, colorless liquid | 6.0 | 3.81 |

The formulations were all manufactured using equipment well-known in the pharmaceutical arts. By way of example, the following process was used to make Formulation Trial No. "33B". Each of the ingredients were weighed under ambient conditions (67 °F to 72 °F, 41% to 56% relative humidity). Methylparaben and propylparaben were dissolved in alcohol, and the resulting solution was added to the first part of water. The mixture was stirred with povidone until it completely dissolved. Dexmedetomidine hydrochloride (a powder) was added to the solution, which was stirred until all of the powder had dissolved. Next, maltitol syrup was added and the solution was stirred until it appeared homogenous. The pH, initially at 3.53, was adjusted by dropwise addition of sodium hydroxide until the pH was 6.99. A total of 5.7 g of sodium hydroxide solution was added. Sufficient water, 38.8 g, was added to bring the total weight to 1000 g.

Human clinical studies have been performed to evaluate the methods and compositions described herein. In one example referred to herein as REC-09-001, a Phase I, single dose, 3-way crossover study was conducted involving 24 healthy male and female subjects. The subjects were divided into four treatment groups involving the following formulations and doses: 1) Formulation DEX-SL.01 = 50 µg (1 pump actuation); 2) Formulation DEX-SL.01 = 100 µg (2 pump actuations); 3) Formulation DEX-SL.02 = 50 µg* (1 pump actuation); and 4) Commercial I.V. - 50 µg over 10 minutes.

**Table 5.**

| **Label Claim (g)** | **w/w %** | **Ingredient** | **Code Number** | **Amount per Batch (g)** |
|---|---|---|---|---|
| | 0.0590 | Dexmedetomidine Hydrochloride | AZP-RM-0320 | 0.59* |
| | 2.000 | Kollidon 25DR (Povidone USP) | AZP-RM-0315 | 20.0 |
| | 10.000 | Malrirol Solurion, NF (MALTISWEET # 3145) | AZP-RM-0317 | 100 |
| | 0.020 | Momosodium Phoasphate, Monohydrate, USP | AZP-RM-169 | 0.20 |
| 0.05 % w/w | 0.500 | Disodium Hydrogen Phosphate Heptahydrate, USP | AZP-RM-168 | 5.0 |
| | 20.84 | Alcohol, USP 190 Proof | AZP-RM-0071 | 208.4 |
| | 10.000 | Diethylene Glycol Monoethyl Ether, NF (Transcutol HP) | AZP-RM-0352 | 100.0 |
| | 50 | Purified Water, USP/EP (Part 1) | AZP-RM-0037 | 500 |
| | QS | Purified Water, USP/EP (Part 2) | AZP-RM-0037 | QS |
| | Qs to adjust pH | 8% w/w Sodium Hydroxide Solution | AZP-RM-0036 | Qs to adjust pH |
| | QS to adjust pH | Hydrocholric Acid Diluted 10%, NF | AZP-RM-0341 | QS to adjust pH |
| | **TOTAL THEORETICAL WEIGHT** | | | **1000g** |

| | | | | |
|---|---|---|---|---|
| *API adjusted according to Relative Density of the liquid media and drug C of A | | | | |

The above Table 5 represents the formulation for DEX-SL.03/.04 where DEX-SL.03 is this formulation provided with a 50 µL spray pump for administration, and DEX-SL.04 is this same formulation administered as drops totaling 100 µL (i.e. 50 µg dose).

The study results showed that in Period 1 of the study (pre-crossover), a significant number of subjects experienced hypotension post-administration, as follows: Subject R006 (100 µg) = Syncope/dizziness episode for approx 2 minutes; Subject R008 (50 µg IV) = Postural hypotension for approx 4 hrs post-dose; Subject R018 (50 µg IV) - Ongoing postural hypotension which required IV saline treatment, resolving 5hrs post-dose; and Subject R021 (100 µg) = Hypotension 1.25hrs post-dose and required IV saline treatment at 4.5 hrs post dose. Sedation was observed in all treatment groups, with sedation in the 100 µg sublingual group observed at a much higher level than in the 50 µg sublingual group.

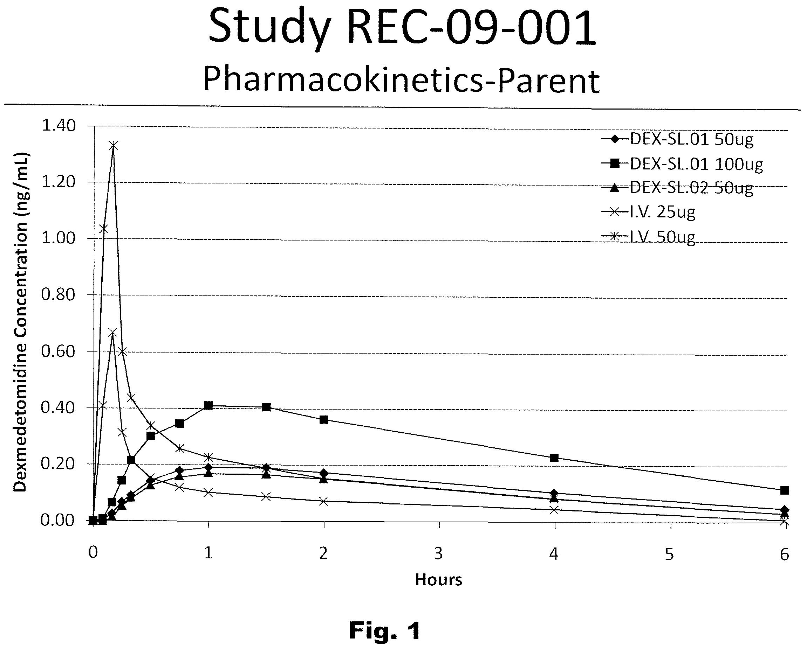

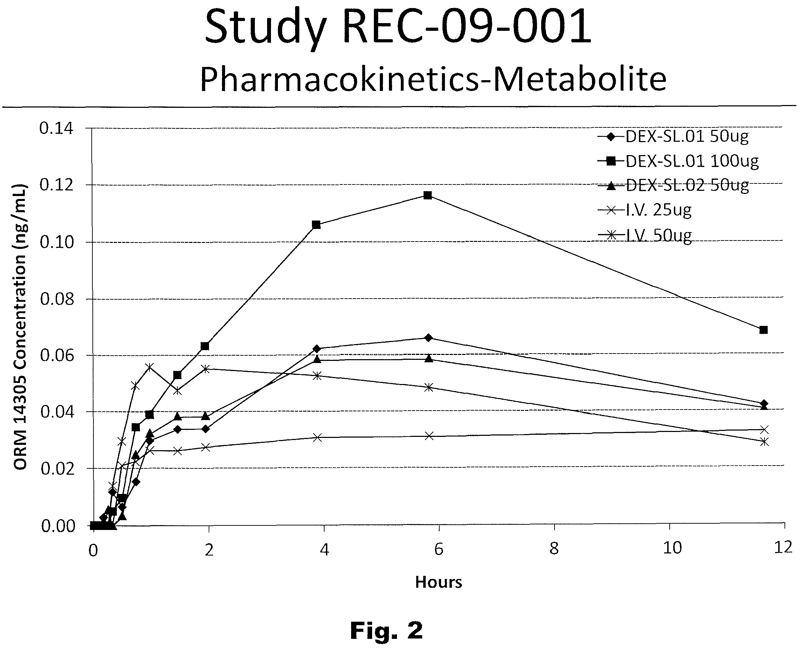

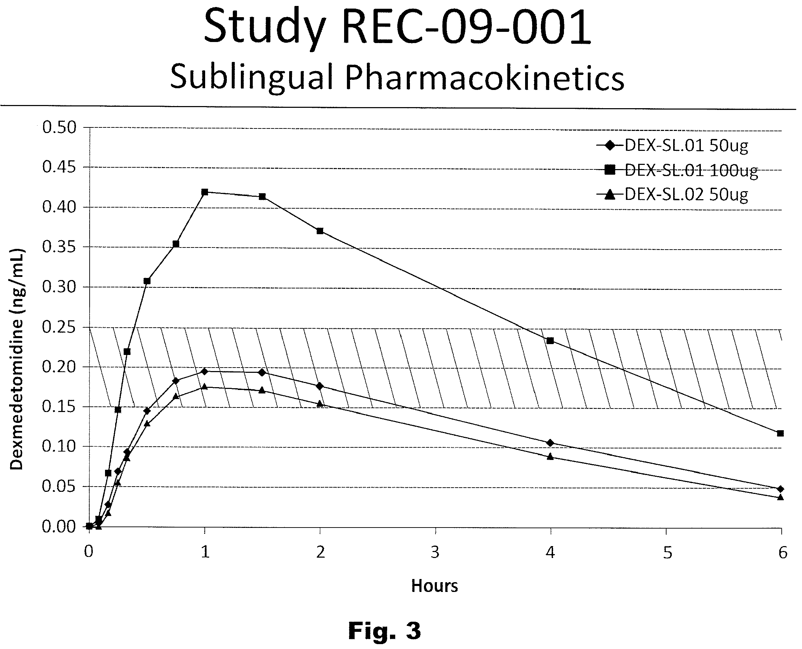

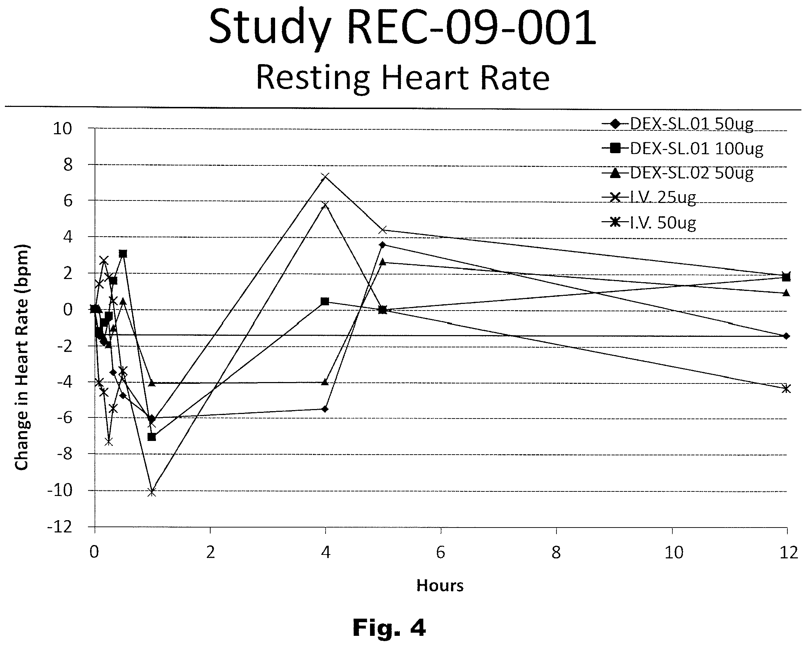

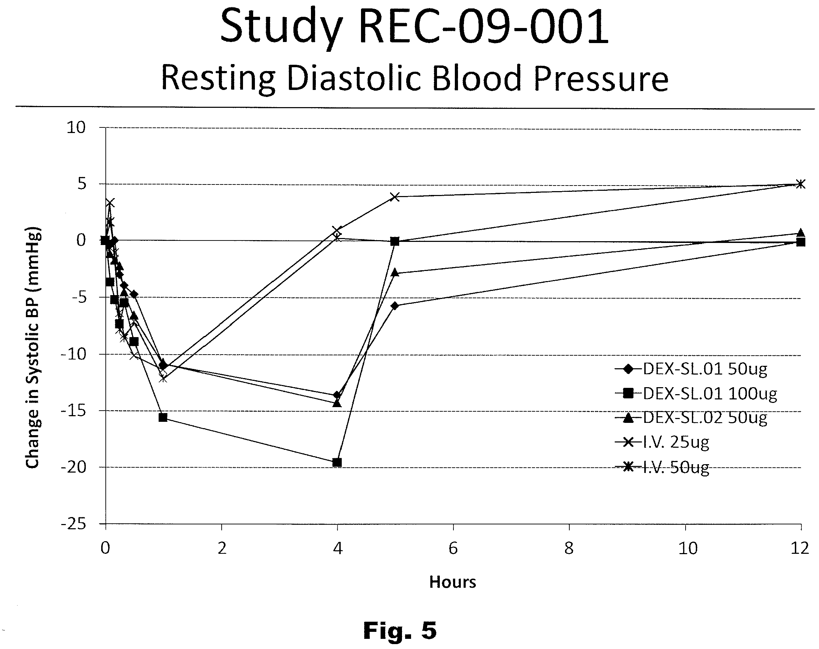

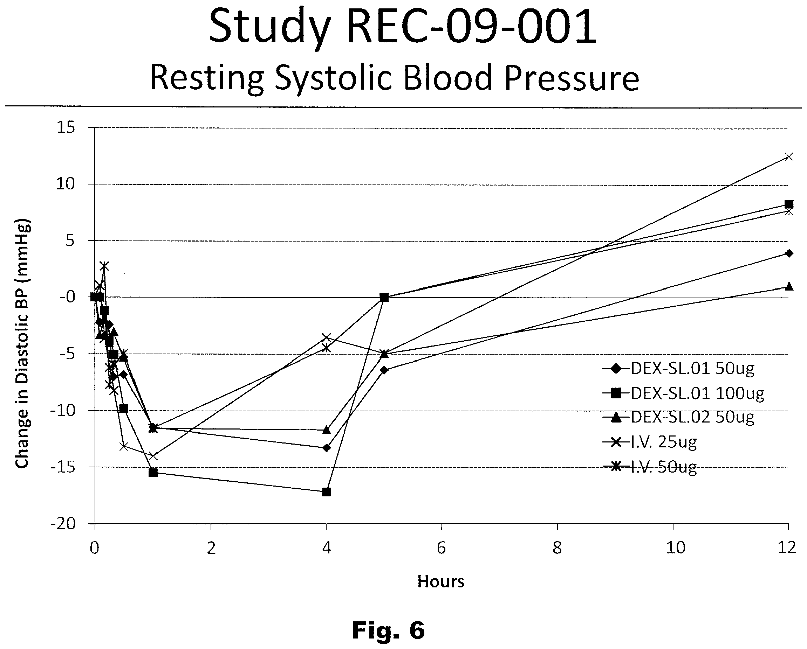

In view of these observations, including side effects as well as the undesirably high level of sedation, the inventors eliminated the 100 µg dose in Period 2 and subsequent Periods of the study. Further, the 50 µg i.v. dose was reduced to 25 µg. The investors also removed the standing vital sign measurement in 1^{st} 4 hours, and added a pre-dose glucose assessment for each subject. Three subjects withdrew prior to Period 2, as follows: Subject R002 for personal reasons; Subject R003 due to AE's experienced from Period 1; and Subject R024 due to pre-dose bradycardia in Period 2. Remaining subjects were then re-randomized to ensure that all subjects received both 50 µg formulations (sublingual sprays). In Period 2, hypotension was again observed, but to a lesser degree than in Period 1. Sedation was also observed in all treatment groups. Other observations from Study REC-09-001 are illustrated in Tables herein, as well as in Figure 1-6.

Notably, as shown in the Figures 1-6, in Study REC-09-001, the 50 µg sublingual formulations achieved a Cₘₐₓ of between about 0.130 to about 0.245 ng/mL, and showed less sedation than the 100 µg sublingual (Cₘₐₓ of between about 0.299 to about 0.574 ng/mL) and less sedation than the 50 µg and 25 µg I.V. formulations (Cₘₐₓ of between about 1.14 to about 1.72 ng/mL, and between about 0.496 and 0.844 ng/mL, respectively. Moreover, the Tₘₐₓ of all sublingual formulations averages about 60 minutes, whereas the I.V. formulations averaged about 10 minutes..

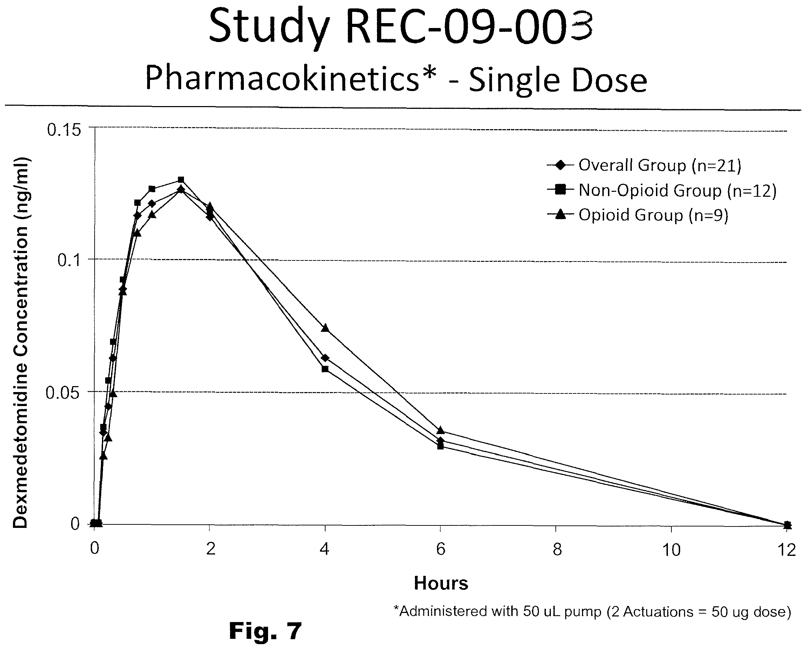

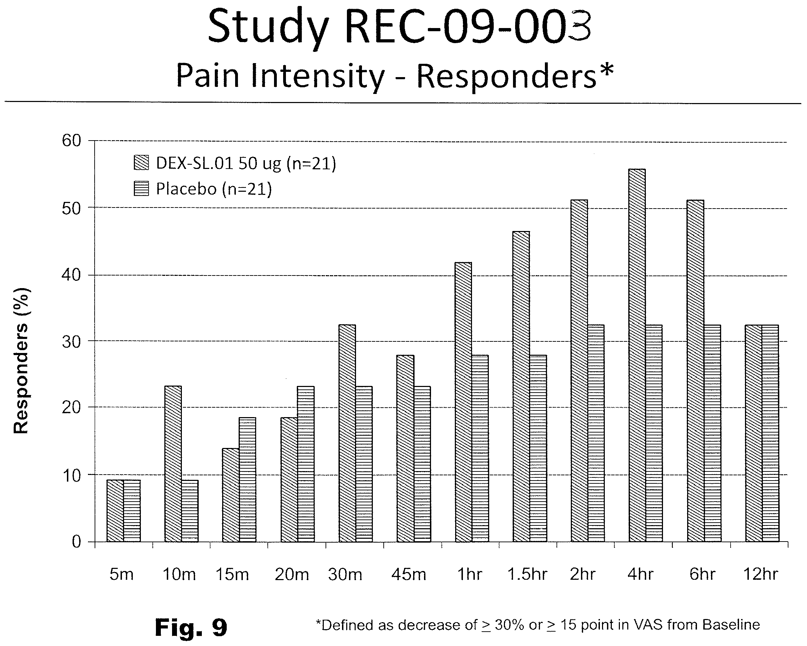

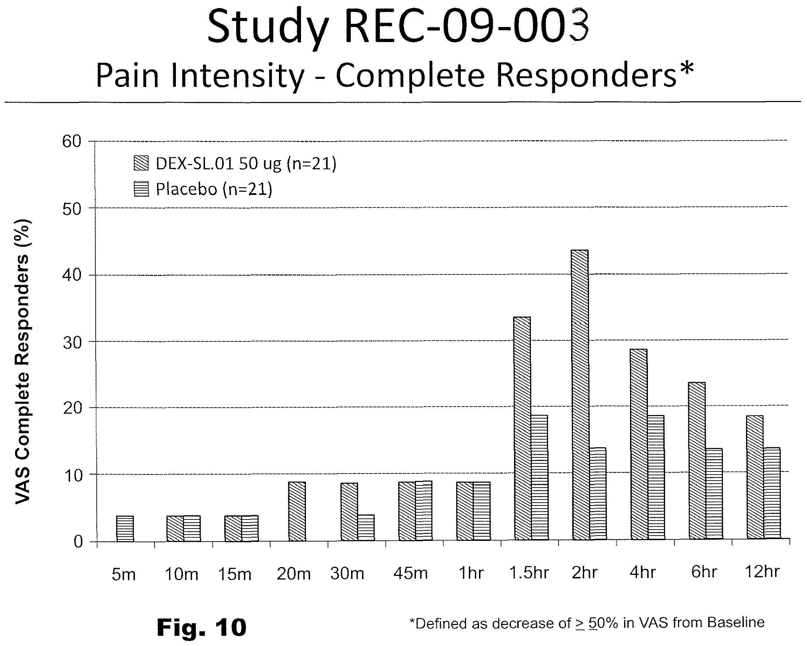

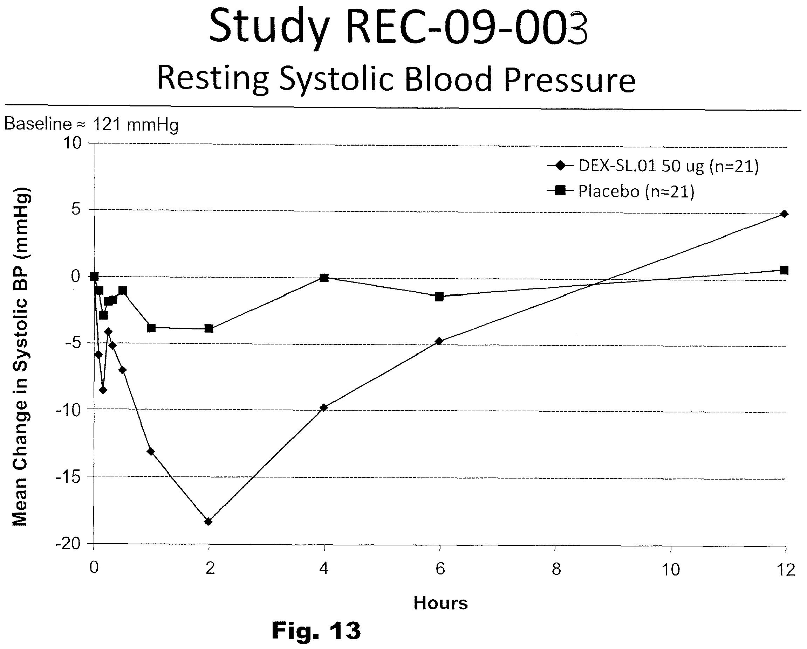

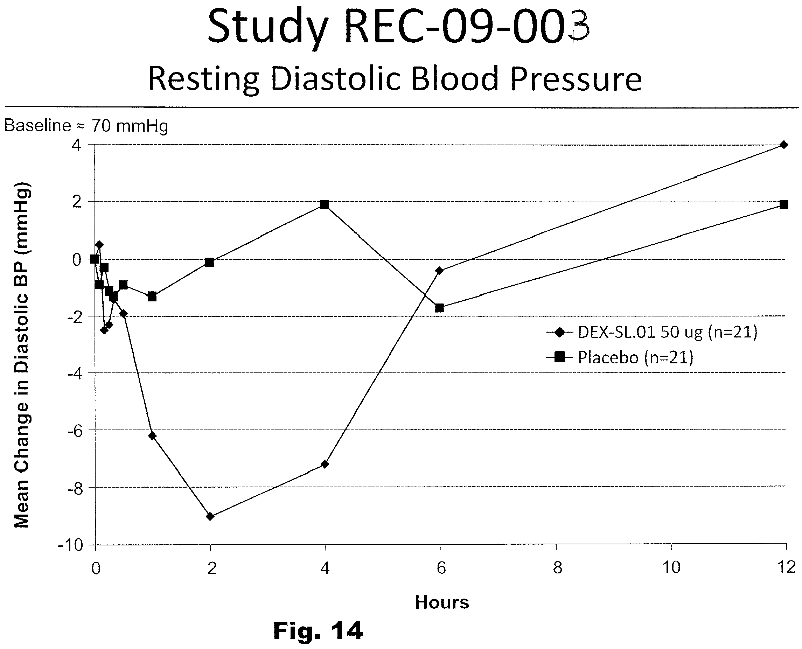

In another example, a human Phase 1 clinical study was conducted, referred to herein as REC-09-004. This study was a single-dose, 3-way complete crossover involving 12 normal healthy subjects. Three formulations containing dexmedetomidine were included, as follows: 1) Old formulation: DEX-SL.01 = 50 µg (Spray - administered with a 50 µL pump so that 2 actuations delivers a 50 µg dose); 2) New formulation: DEX-SL.03 - 50 µg (Spray-administered with a 50 µL pump so that 2 actuations delivers a 50 µg dose); and 3) New formulation: DEX-SL.04 - 50 µg (Drops - administered under the tongue). However, the pharmacokinetic ("PK") results from this patient population were found to be inconsistent compared with those of Study REC-09-001. Thus, two additional treatments were added to provide additional data and to evaluate possible effects of two suspected variable factors, namely the use of a 100 µL vs. 50 µL pump, and the effect of mouth pH. Thus, 8 subjects were made available for two additional periods of study to receive : 4) DEX-SL.01 - 50 µg (administered with 100 µL pump to provide the 50 µg dose in one actuation); and 5) DEX-SL.01 - 50 µg (Following oral pH buffering to ∼8.0) The results of the study are illustrated in Figures ***7-14***. As shown, for each formulation, the concentration of dexmedetomidine peaked within 2 hours, reaching a peak Cmax of less than 0.15 ng/mL, and then decreasing slowly but maintaining a Cmax of greater than 0.05 through at least hour 5.

In another example, another human Phase 1 clinical study was conducted, referred to herein as REC-09-003. That study involved 24 chronic lower back pain (CLBP) patients; 12 were non-opioid users, and the other 9 were opioid users. The study was divided into two parts. Part 1 was designed to evaluate blood pressure, heart rate and sedation effects in a pain state, and also to evaluate analgesia. In Part 1, the formulation administered was DEX-SL.01 50 µg (Administered with a 50 µL pump (2 actuations = 50 µg dose); compared to placebo. Part 2 was designed to evaluate the safety of multiple doses (q. 6 hours), and also the effects of dexmedetomidine with concomitant opioids. Again, the formulation administered was DEX-SL.01 50 µg (administered with a 50 µL pump (2 actuations = 50 µg dose). The analgesic effect was measured using: 1) a pain intensity score involving a visual analog scale (range 0 to 100), wherein 0 represents no pain, and 100 represents the worst imaginable pain; and 2) a pain relief score, wherein 0 represents none, 1 represents slight relief, 2 represents moderate relief, 3 represents lots of relief, and 4 represents complete relief.

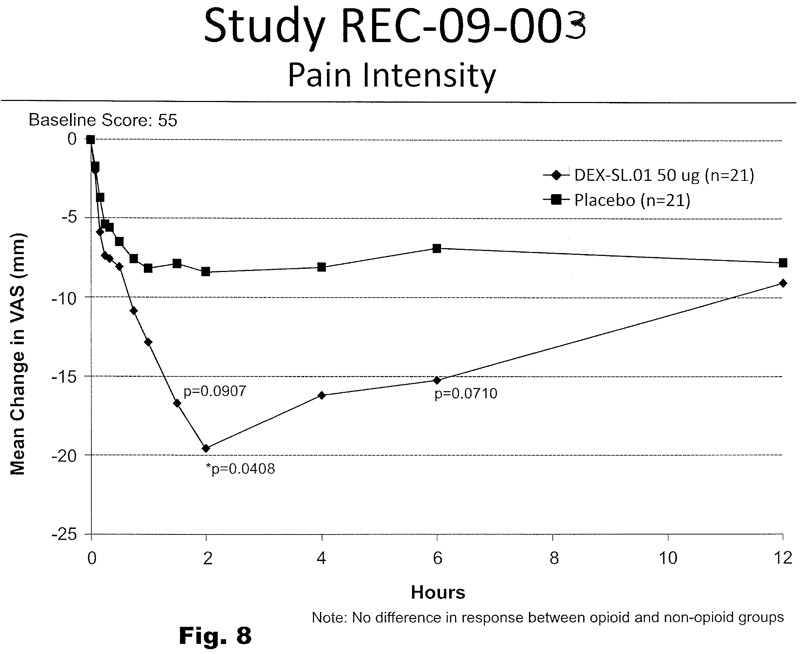

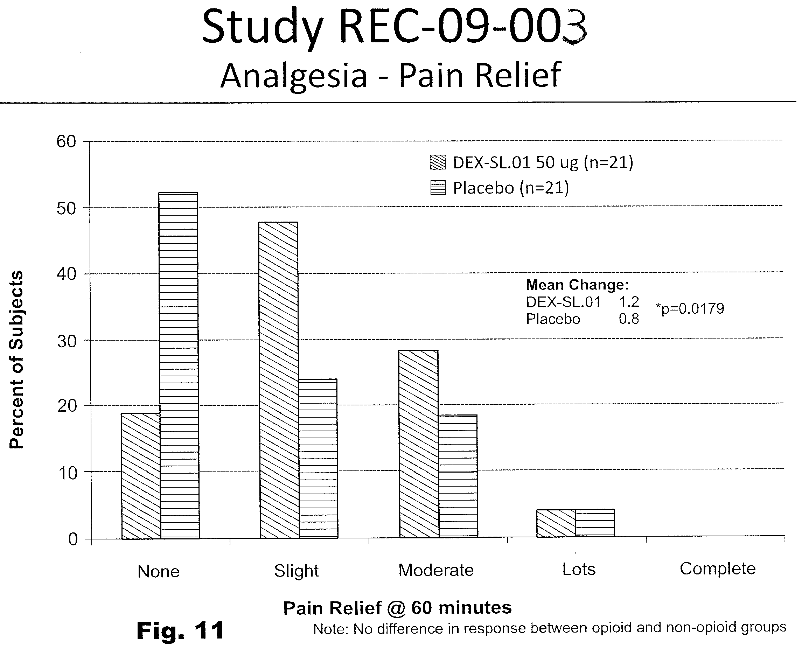

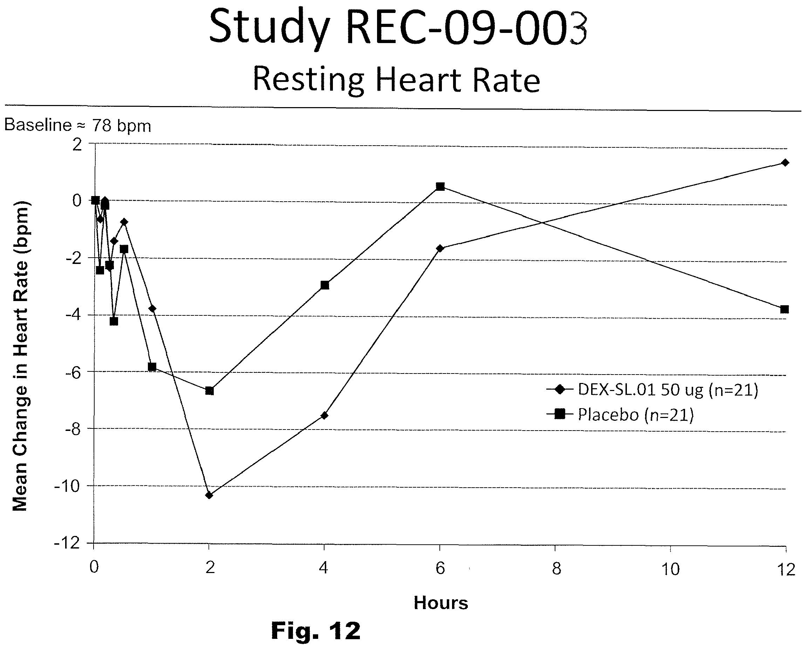

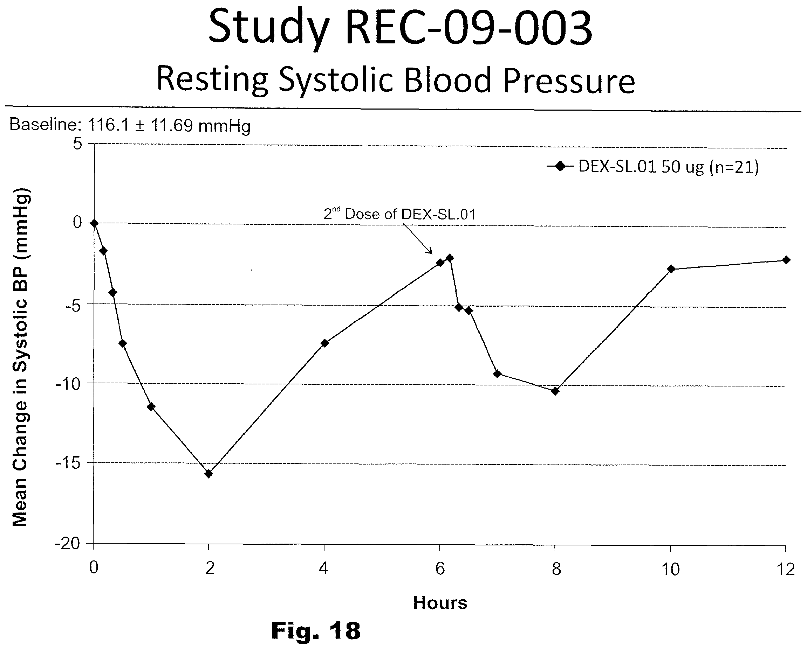

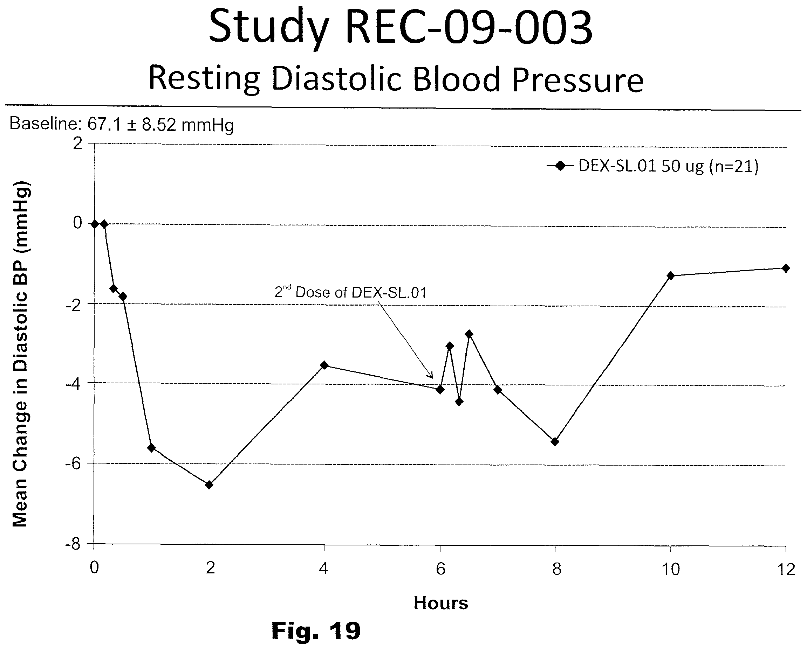

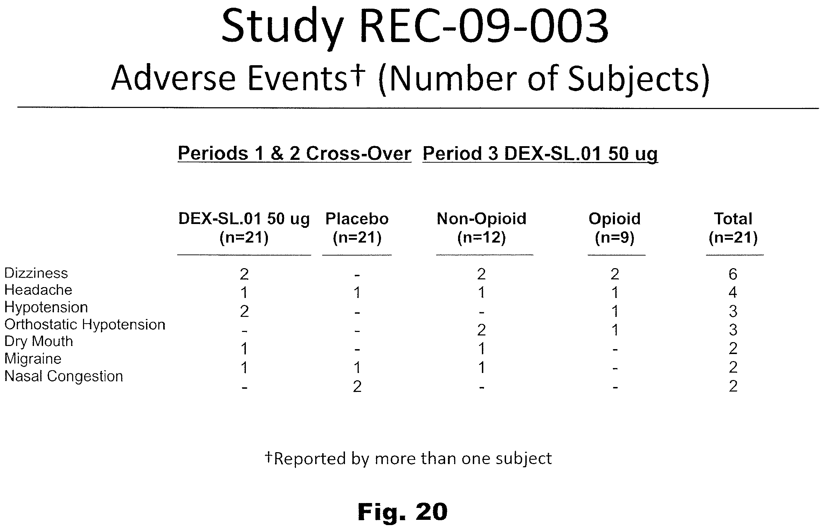

The results of the study REC-09-003 are graphically depicted in Figures 7-20. Those figures collectively illustrate the pharmacokinetics of the single dose and multiple doses. Figure 7 illustrates the pharmacokinetcis of single dose in Part 1, wherein no difference was observed in response between opioid and non-opioid patient groups. As shown in Figs. 8-10, significant differences in pain intensity were observed between the drug formulation and placebo. For Part 1, Figure 7 illustrates the observed results in pain intensity, analgesia and pain relief. Figures 11 illustrates the observed results concerning analgesia, while Figures 12-14 illustrate Part 1 observations for resting heart rate, resting systolic blood pressure, and resting diastolic blood pressure, respectively.

With respect to sedation in Study REC-09-003, measurements were made by both the investigator and the patients. In the case of the investigator, the Ramsay Sedation Scale was applied, as follows: Level 1 = anxious, agitated, or restless;; Level 2 = cooperative, oriented, and tranquil; Level 3 = sedated but responds to commands; Level 4 = asleep; brisk response to light glabellar tap or loud auditory stimulus; Level 5 = asleep; sluggish response to light glabellar tap or loud auditory stimulus; Level 6 = asleep; no response to painful stimulus.

With respect to patient subjects in study REC-09-003, the Stanford Sleepiness Scale was applied, with Subject patients rating their degree of sedation as: Level 1 = Feeling active, vital, alert, or wide awake; Level 2 = Functioning at high levels, but not at peak; able to concentrate; Level 3 = Awake, but relaxed; responsive but not fully alert; Level 4 = Somewhat foggy, let down; Level 5 = Foggy; losing interest in remaining awake; slowed down; Level 6 = Sleepy, woozy, fighting sleep; prefer to lie down; or Level 7 No longer fighting sleep, sleep onset soon; having dream-like thoughts.

Notably, on the Ramsay Sedation Scale, all subjects rated "2" at baseline and 12 hours, with Peak Sedation achieved wherein 43% of DEX-SL.01 subjects were rated a Level 3 at 2 hours. Notably, only 1 DEX-SL.01 subject rated greater than a Level 3 at any time. With respect to patient self-assessment on the Stanford Sleepiness Scale, all subjects rated less than or equal to a Level 3 at baseline. Peak Sleepiness was experienced in 69% of DEX-SL.01 subjects as represented by a self-rating of greater than 3 at 2 hours.

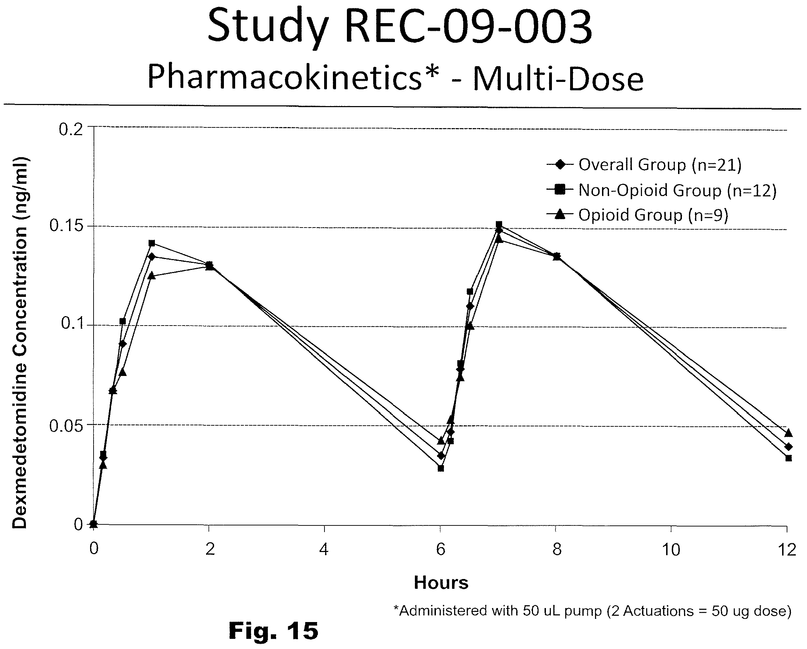

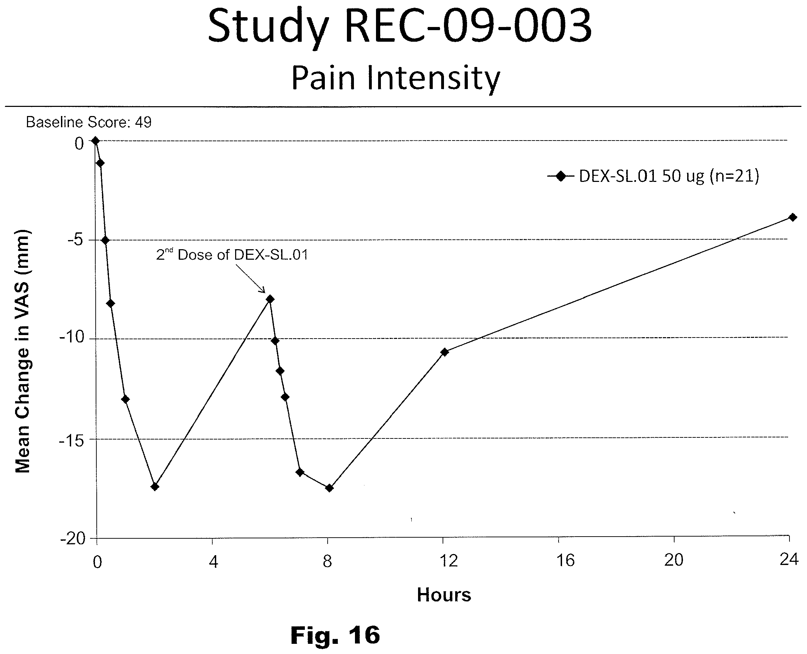

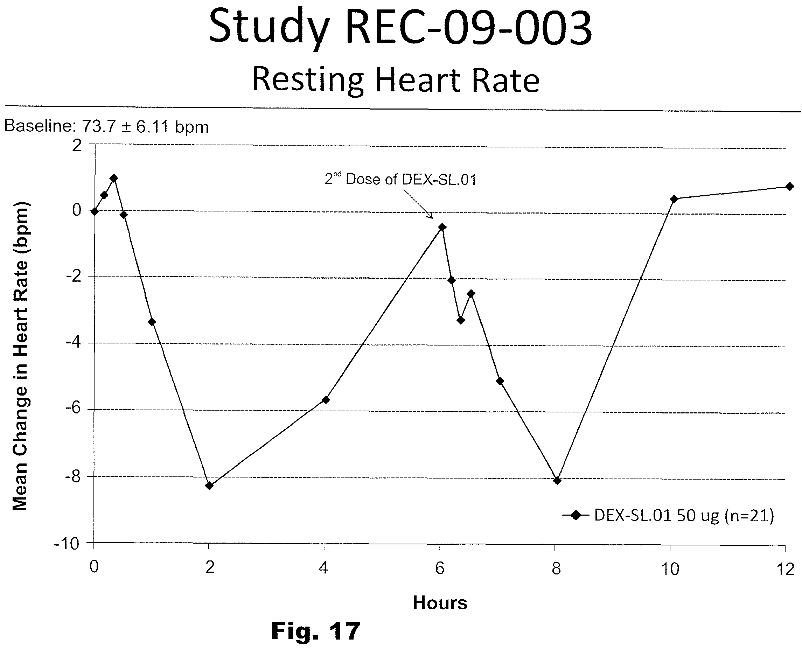

In Part 2 of Study REC-09-003, the effect of multiple doses of the formulation was evaluated. As illustrated in Figures 15-19, the pharmacokinetics and effects of a multi-dose of formulation DEX-SL.01 at q. 6 hours was similar among the opioid and non-opioid patent subjects, with peak blood concentrations occurring at between about 1 to about 2 hours, and again at about 7 to about 7.5 hours. Notably, the blood concentrations were maintained above 0.05 ng/mL for about 5 hours after each dose - with profiles exhibiting strong similarity to that of the single dose pharmacokinetics. As shown in Figure 16, pain intensity of the multiple dose regimen of q. 6 hours was also evaluated. The peak decrease in pain intensity occurred at approximately 2 hours after each dose, essentially consistent with the Cmax peaks shown in the pharmacokinetics of Figure 15. Similarly, as shown in Figures 17-19, the effect of multiple doses on resting heart rate, resting systolic blood pressure, and resting diastolic blood pressure was evaluated, with peak changes occurring consistent with the peak Cmax of each dose. A rebound blood pressure pattern/spike was sometimes associated with the second dose (shortly after q. 6 hours), as shown in Figure 17.

While this description is made with reference to exemplary embodiments, it will be understood by those skilled in the art that various changes may be made and equivalents may be substituted for elements thereof without departing from the scope. In addition, many modifications may be made to adapt a particular situation or material to the teachings hereof without departing from the essential scope. Also, in the description there have been disclosed exemplary embodiments and, although specific terms may have been employed, they are unless otherwise stated used in a generic and descriptive sense only and not for purposes of limitation, the scope of the claims therefore not being so limited. Moreover, one skilled in the art will appreciate that certain steps of the methods discussed herein may be sequenced in alternative order or steps may be combined. Therefore, it is intended that the appended claims not be limited to the particular embodiment disclosed herein.

Preferred embodiments of the invention are as follows:
1. A method of treating or preventing pain comprising
   applying to the oral mucosa of a human a pharmaceutical composition comprising a dosage of dexmedetomidine or a pharmaceutically acceptable salt thereof in a pharmaceutically acceptable liquid vehicle,
   wherein said dexmedetomidine or pharmaceutically acceptable salt thereof is absorbed through said oral mucosa and produces analgesia without sedation.
2. The method according to paragraph 1, wherein said dosage of dexmedetomidine or pharmaceutically acceptable salt thereof is between about 0.05 µg/kg and about 1.50 µg/kg.
3. The method according to paragraph 1, wherein said human is an adult, and said dosage of dexmedetomidine or pharmaceutically acceptable salt thereof is between about 5 µg and about 50 µg.
4. The method according to paragraph 1, wherein the plasma Cₘₐₓ of dexmedetomidine upon transmucosal absorption into the systemic circulatory system of said human is less than about 0.30 ng/mL.
5. The method according to paragraph 1, wherein said applying step comprises sublingually or buccally applying said pharmaceutical composition to the oral cavity of said human.
6. The method according to paragraph 1, wherein said applying step comprises applying drops of said pharmaceutical composition to said oral mucosa of said human.
7. The method according to paragraph 1, wherein said applying step comprises spraying said pharmaceutical composition into the oral cavity of said human.
8. The method according to paragraph 1, wherein, during the hour immediately after applying said pharmaceutical composition, the resting mean arterial blood pressure varies by no more than about 20 mmHg.
9. The method according to paragraph 1, wherein, during the hour immediately after applying said, said human is able to remain awake and to respond to commands, and said human is alert and oriented.
10. The method according to paragraph 1, wherein said dexmedetomidine or pharmaceutically acceptable salt thereof is co-administered with one or more other analgesics.
11. The method according to paragraph 10, wherein said pharmaceutically effective amount pharmaceutical composition is administered every 6 hours intermittently to treat breakthrough pain that is inadequately controlled by said one or more other analgesics.
12. The method according to paragraph 1, wherein said pharmaceutically effective amount pharmaceutical composition is self administered by said human.
13. The method according to paragraph 1, wherein said pharmaceutical composition is administered to said human by a caregiver.
14. The method according to paragraph 1, wherein said pain is idiopathic pain.
15. The method according to paragraph 14, wherein said idiopathic pain is selected from the group consisting of neuralgia, myalgia, hyperalgia, hyperpathia, neuritis, and neuropathy.
17. The method according to paragraph 1, wherein said pain is associated with or caused by cancer, viral infection, physical trauma, arthritis, headache, or lower back pain.
18. The method according to paragraph 17, wherein said physical trauma is associated with or caused by surgery, a burn, or blunt force trauma.
19. A method of treating or preventing pain comprising
   applying to a mucous membrane of a mammal a pharmaceutical composition comprising dexmedetomidine, or a pharmaceutically acceptable salt or pro-drug thereof, in a pharmaceutically acceptable liquid vehicle,
   wherein said dexmedetomidine, or said pharmaceutically acceptable salt or pro-drug thereof, is absorbed through said mucous membrane and produces analgesia without sedation.
20. A method of treating or preventing pain comprising
   administering to the oral mucosa of a mammal a systemically absorbed pharmaceutical composition comprising dexmedetomidine, or a pharmaceutically acceptable salt or pro-drug thereof, in an amount effective to treat or to prevent pain in said mammal upon administration,
   wherein said pharmaceutical composition provides a physiologically active amount of dexmedetomidine into the systemic circulatory system of said mammal at a rate that produces an analgesic effect without sedation within one hour of administration.
21. An analgesic pharmaceutical composition comprising dexmedetomidine, or a pharmaceutically acceptable salt or pro-drug thereof, in and a pharmaceutically acceptable liquid vehicle, said pharmaceutical composition being configured and adapted for transmucosal administration to a mammal by applying said analgesic pharmaceutical composition to a mucous membrane of said mammal.
22. The analgesic pharmaceutical composition according to paragraph 21, wherein said analgesic pharmaceutical composition is configured and adapted for sublingual administration by applying said composition to a mucous membrane under the tongue of said mammal.
23. The analgesic pharmaceutical composition according to paragraph 21, wherein said analgesic pharmaceutical composition is configured and adapted for buccal administration by applying said composition to a mucous membrane of a buccal cavity of said mammal.
24. The analgesic pharmaceutical composition according to paragraph 21, wherein said analgesic pharmaceutical composition is configured and adapted for transmucosal administration by applying drops of said composition to a mucous membrane within oral cavity of said mammal.
25. The analgesic pharmaceutical composition according to paragraph 21, wherein said analgesic pharmaceutical composition is configured and adapted for transmucosal administration by applying a spray of said composition to a mucous membrane within oral cavity of said mammal.
26. The analgesic pharmaceutical composition according to paragraph 21, wherein said pharmaceutically acceptable salt of dexmedetomidine is dexmedetomidine hydrochloride, and wherein said liquid vehicle is an aqueous solution of said dexmedetomidine hydrochloride.
27. The analgesic pharmaceutical composition according to paragraph 21, wherein said analgesic pharmaceutical composition is packaged in a dispensing device.
28. An apparatus for treating or preventing pain, said apparatus comprising
   an analgesic pharmaceutical composition comprising dexmedetomidine, or a pharmaceutically acceptable salt or pro-drug thereof, in a pharmaceutically acceptable liquid vehicle, and a dispensing device that contains and dispenses said analgesic pharmaceutical composition.
29. The apparatus according to paragraph 28, wherein said dispensing device is configured to deliver a unit dosage of between about 25 µL and about 200 µL of said analgesic pharmaceutical composition.
30. The apparatus according to paragraph 28, wherein said dispensing device is configured to deliver a unit dosage of between about 50 µL and about 100 µL of said analgesic pharmaceutical composition.
31. The apparatus according to paragraph 28, wherein said dispensing device is configured to delivers a unit dosage of between about 5 µg and about 50 µg of said dexmedetomidine, or said pharmaceutically acceptable salt or pro-drug thereof.

## Claims

1. A composition comprising dexmedetomidine or a pharmaceutically acceptable salt thereof, povidone, propylparaben, methylparaben, maltitol, ethanol, and water.

2. The composition according to claim 1 wherein the dexmedetomidine is dexmedetomidine hydrochloride.

3. The composition according to claim 1 or claim 2 wherein the maltitol is maltitol syrup.

4. The composition according to any one of claims 1 to 3 further comprising citric acid and sodium citrate.

5. The composition according to claim 4 wherein the citric acid is anhydrous citric acid, and/or wherein the sodium citrate is sodium citrate dihydrate.

6. The composition according to any one of claims 1 to 5 wherein the dexmedetomidine is present at a concentration of 500 µg/ml.

7. A method of preparing the composition according to any one of claims 1 to 6 comprising the steps:
dissolving methylparaben and propylparaben in ethanol to form a first solution;
adding the first solution to water to form a second solution;
adding povidone to the second solution until it is completely dissolved to form a third solution;
adding dexmedetomidine powder to the third solution until all of the powder is dissolved to form a fourth solution; and
adding maltitol to the fourth solution to form a fifth solution, and stirring the fifth solution until it is homogenous.

8. The method according to claim 7 further comprising adjusting the pH of the fifth solution until it is neutral.

9. The method according to claim 8 further comprising adding water to the fifth solution.

10. The method according to any one of claims 7 to 9 further comprising adding citric acid and sodium citrate to the composition.

11. The composition according to any one of claims 1 to 6 for use in the treatment or prevention of pain in a human, wherein during the hour immediately after administration of the composition, the human is able to remain awake and to respond to commands and the human is alert and oriented, wherein said composition is for administration to the oral mucosa of the human and wherein the unit dosage of dexmedetomidine, or pharmaceutically acceptable salt thereof, is between about 50 µl and about 100 µl.

12. The composition for use according to claim 11, **characterized in that**,
(a) the composition is administered sublingually or buccally to the oral cavity of the human, or
(b) the composition is co-administered with one or more other analgesics.

13. The composition for use according to claim 11, **characterized in that** the pain is idiopathic pain, neuralgia pain, myalgia pain, hyperalgia pain, hyperpathia pain, neuritis pain, neuropathic pain, pain associated with or caused by cancer, pain associated with or caused by viral infection, pain associated with or caused by physical trauma, pain associated with or caused by arthritis, headache pain, lower back pain, pain associated with or caused by surgery, pain associated with or caused by a burn, or pain associated with or caused by blunt force trauma.

14. The composition for use according to claim 11, **characterized in that** the pharmaceutically acceptable salt of dexmedetomidine is dexmedetomidine hydrochloride.

15. A composition for use in the treatment or prevention of pain in a human, wherein during the hour immediately after administration of the composition, the human is able to remain awake and to respond to commands and the human is alert and oriented, **characterized in that** the composition comprises dexmedetomidine, or a pharmaceutically acceptable salt thereof, in a pharmaceutically acceptable liquid vehicle, wherein said composition is for administration to the oral mucosa of the human and wherein the dosage of dexmedetomidine, or pharmaceutically acceptable salt thereof is between 5 µg and 50 µg.
